# EUROPEAN PATENT APPLICATION

(11) **EP 4 044 063 A1**
(43) Date of publication of application: **17.08.2022**
(21) Application number: 20887729.0
(22) Date of filing: 10.11.2020
(51) Int. Cl.: G06F 30/27, G06F 30/10

(54) **INFORMATION PROCESSING SYSTEM, INFORMATION PROCESSING METHOD, AND INFORMATION PROCESSING PROGRAM**

(30) Priority: 11.11.2019 JP 2019204086
(71) Applicant: Showa Denko Materials Co., Ltd., Tokyo 100-6606 (JP)
(72) Inventor: HANAOKA, Kyohei, Tokyo 100-6606 (JP)
(74) Representative: Berggren Oy
(86) International application number: PCT/JP2020/041902
(87) International publication number: WO 2021/095722

(57) **Abstract**

An information processing system according to an embodiment is configured to: acquire a numerical representation and a combination ratio for each of a plurality of component objects; calculate a first feature vector of each of the plurality of component objects by inputting a plurality of numerical representations corresponding to the plurality of component objects into a first machine learning model; calculate a second feature vector of each of the plurality of component objects by applying the combination ratio to the first feature vector for each of the plurality of component objects; and calculate a composite feature vector indicating features of a composite object obtained by combining the plurality of component objects, by inputting a plurality of second feature vectors corresponding to the plurality of component objects into a second machine learning model.

## Description

### Technical Field

One aspect of the present disclosure relates to an information processing system, an information processing method, and an information processing program.

### Background Art

A method of analyzing a composite object obtained by combining a plurality of component objects using machine learning has been used. For example, Patent Literature 1 describes a method of predicting the bondability between the three-dimensional structure of a biopolymer and the three-dimensional structure of a compound. This method includes: generating a predicted three-dimensional structure of a complex of a biopolymer and a compound based on the three-dimensional structure of the biopolymer and the three-dimensional structure of the compound; converting the predicted three-dimensional structure into a predicted three-dimensional structure vector representing a result of comparison with an interaction pattern; and predicting the bondability between the three-dimensional structure of the biopolymer and the three-dimensional structure of the compound by determining the predicted three-dimensional structure vector using a machine learning algorithm.

### Citation List

### Patent Literature

Patent Literature 1: JP 2019-28879 A

### Summary of Invention

### Technical Problem

When there are various or many component objects, it is not possible to prepare a sufficient amount of data for these component objects. As a result, the accuracy of analysis of a composite object may not reach the expected level. Therefore, there has been a demand for a mechanism for improving the accuracy of analysis of a composite object even in a case where a sufficient amount of data cannot be prepared for component objects.

### Solution to Problem

An information processing system according to one aspect of the present disclosure includes at least one processor. The at least one processor is configured to: acquire a numerical representation and a combination ratio for each of a plurality of component objects; calculate a first feature vector of each of the plurality of component objects by inputting a plurality of the numerical representations corresponding to the plurality of component objects into a first machine learning model; calculate a second feature vector of each of the plurality of component objects by applying the combination ratio to the first feature vector for each of the plurality of component objects; calculate a composite feature vector indicating features of a composite object obtained by combining the plurality of component objects, by inputting a plurality of the second feature vectors corresponding to the plurality of component objects into a second machine learning model; and output the composite feature vector.

In such an aspect, the features of each component object configuring the composite object are clarified before considering the combination ratio. It is therefore possible to improve the accuracy of analysis of composite object even in a case where a sufficient amount of data cannot be prepared for the component objects.

### Advantageous Effects of Invention

According to one aspect of the present disclosure, it is possible to improve the accuracy of analysis of composite object even in a case where a sufficient amount of data cannot be prepared for the component objects.

### Brief Description of Drawings

FIG. 1 is a diagram showing an example of the hardware configuration of a computer configuring an information processing system according to an embodiment.
FIG. 2 is a diagram showing an example of the functional configuration of the information processing system according to the embodiment.
FIG. 3 is a flowchart showing an example of an operation of the information processing system according to the embodiment.
FIG. 4 is a diagram describing the operation of the information processing system according to the embodiment by using a specific example of data.

### Description of Embodiments

Hereinafter, an embodiment in the present disclosure will be described in detail with reference to the accompanying diagrams. In addition, in the description of the diagrams, the same or equivalent elements are denoted by the same reference numerals, and the repeated description thereof will be omitted.

### [System overview]

An information processing system 10 according to the embodiment is a computer system that performs an analysis on a composite object obtained by combining a plurality of component objects at a predetermined combination ratio. A component object refers to a tangible object or an intangible object used to generate a composite object. The composite object can be a tangible object or an intangible object. Examples of a tangible object include any substance or object. Examples of an intangible object include data and information. "Combining a plurality of component objects" refers to a process of making a plurality of component objects into one object, that is, a composite object. The method of combining is not limited, and may be, for example, compounding, blending, synthesis, bonding, mixing, merging, combination, chemical combination, or uniting, or other methods. The analysis of a composite object refers to a process for obtaining data indicating a certain feature of the composite object.

The plurality of component objects may be any plurality of types of materials. In this case, the composite object is a multi-component substance produced by these materials. The materials are arbitrary components used to produce a multi-component substance. For example, the plurality of materials may be any plurality of types of molecules or atoms. In this case, the composite object is a multi-component substance obtained by combining these molecules or atoms using an arbitrary method. For example, the material may be a polymer and correspondingly the multi-component substance may be a polymer alloy. The material may be a monomer and correspondingly the multi-component substance may be a polymer. The material may be a medicinal substance, that is, a chemical substance having a pharmacological action, and correspondingly, the multi-component substance may be a medicine.

The information processing system 10 performs machine learning for the analysis of a composite object. The machine learning is a method of autonomously finding a law or rule by learning based on given information. The specific method of machine learning is not limited. For example, the information processing system 10 may perform machine learning using a machine learning model that is a calculation model configured to include a neural network. The neural network is an information processing model that imitates the mechanism of the human cranial nerve system. As a more specific example, the information processing system 10 may perform machine learning by using at least one of graph neural network (GNN), convolutional neural network (CNN), recurrent neural network (RNN), attention RNN, and multi-head attention.

### [System configuration]

The information processing system 10 is configured to include one or more computers. In a case where a plurality of computers are used, one information processing system 10 is logically constructed by connecting these computers to each other through a communication network, such as the Internet or an intranet.

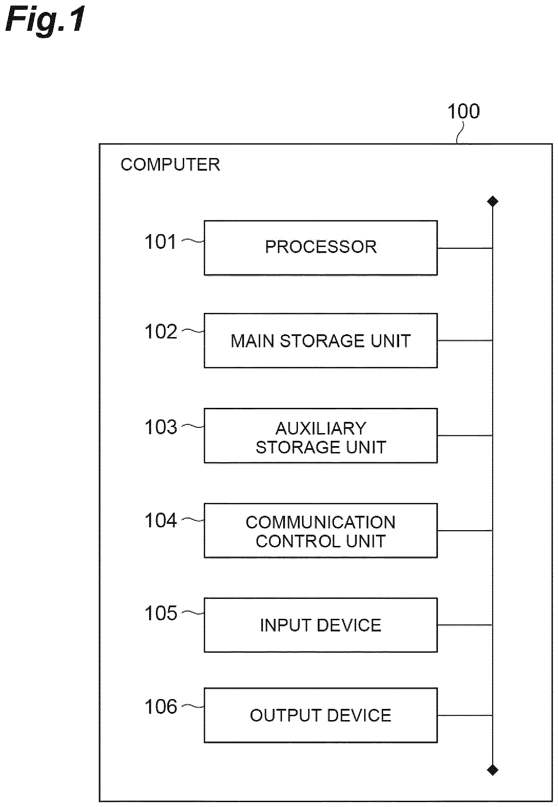

FIG. 1 is a diagram showing an example of a general hardware configuration of a computer 100 configuring the information processing system 10. For example, the computer 100 includes a processor (for example, a CPU) 101 for executing an operating system, an application program, and the like, a main storage unit 102 configured by a ROM and a RAM, an auxiliary storage unit 103 configured by a hard disk, a flash memory, and the like, a communication control unit 104 configured by a network card or a wireless communication module, an input device 105 such as a keyboard and a mouse, and an output device 106 such as a monitor.

Each functional element of the information processing system 10 is realized by reading a predetermined program on the processor 101 or the main storage unit 102 and causing the processor 101 to execute the program. The processor 101 operates the communication control unit 104, the input device 105, or the output device 106 according to the program and performs reading and writing of data in the main storage unit 102 or the auxiliary storage unit 103. The data or database required for the processing is stored in the main storage unit 102 or the auxiliary storage unit 103.

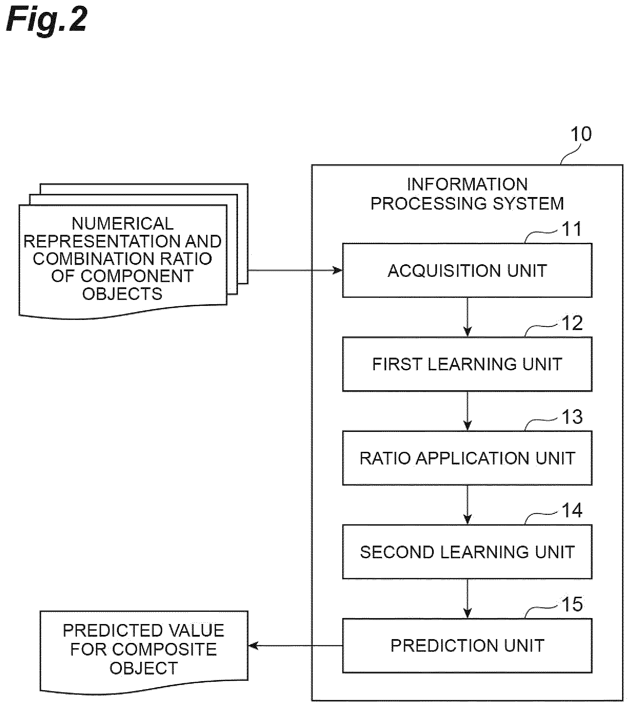

FIG. 2 is a diagram showing an example of the functional configuration of the information processing system 10. The information processing system 10 includes an acquisition unit 11, a first learning unit 12, a ratio application unit 13, a second learning unit 14, and a prediction unit 15 as functional elements.

The acquisition unit 11 is a functional element for acquiring data relevant to a plurality of component objects. Specifically, the acquisition unit 11 acquires a numerical representation and a combination ratio for each of the plurality of component objects. The numerical representation of a component object refers to data representing arbitrary attributes of the component object using a plurality of numerical values. The attributes of the component object refer to the properties or features of the component object. The numerical representation may be visualized by various methods. For example, the numerical representation may be visualized by methods such as numbers, letters, texts, molecular graphs, vectors, images, time-series data, and the like or may be visualized by any combination of two or more of these methods. Each numerical value that makes up the numerical representation may be represented in decimal or may be represented in other notations such as a binary notation and a hexadecimal notation. The combination ratio of component objects refers to a ratio between a plurality of component objects. The specific type, unit, and representation method of the combination ratio are not limited, and may be arbitrarily determined depending on the component object or the composite object. For example, the combination ratio may be represented by a ratio such as a percentage or by a histogram, or may be represented by an absolute amount of each component object.

The first learning unit 12 is a functional element that calculates a first feature vector, which is a vector indicating features of a component object, for each of a plurality of component objects by first machine learning using a first machine learning model. The features of a component object refer to arbitrary elements that make the component object different from other objects. The vector refers to an n-dimensional quantity having n numerical values, and may be expressed as a one-dimensional array.

The ratio application unit 13 is a functional element that calculates a second feature vector of each of the plurality of component objects by applying the combination ratio to the first vector for each of the plurality of component objects.

The second learning unit 14 is a functional element that calculates a composite feature vector, which is a vector indicating features of a composite object, by second machine learning using a second machine learning model. The features of the composite object refer to arbitrary elements that make the composite object different from other objects.

The prediction unit 15 is a functional element that predicts characteristics of the composite object and outputs the predicted value. The characteristics of the composite object refer to unique properties of the composite object.

In one example, both the first and second machine learning models are trained models that are expected to have the highest estimation accuracy, and therefore can be referred to as "best machine learning models". However, it should be noted that the trained model is not always "best in reality". The trained model is generated by processing training data including many combinations of input vectors and labels with a given computer. The given computer calculates an output vector by inputting the input vector into the machine learning model, and obtains an error between a predicted value obtained from the calculated output vector and a label indicated by training data (that is, a difference between the estimation result and the ground truth). Then, the computer updates a predetermined parameter in the machine learning model based on the error. The computer generates a trained model by repeating such learning. The computer that generates a trained model is not limited, and may be, for example, the information processing system 10 or another computer system. The process of generating the trained model can be referred to as a learning phase, and the process of using the trained model can be referred to as an operation phase.

### [Data]

As described above, each component object may be a material, and the composite object may be a multi-component substance. In this case, the numerical representation of the component object (material) may include a numerical value indicating the chemical structure of the material, or may include a numerical value indicating a configuration repetition unit (CRU) of the chemical structure of the material. The combination ratio may be a compounding ratio or a mixing ratio. The predicted value of the characteristics of the composite object (multi-component substance) may indicate at least one of the glass transition temperature (Tg) and elastic modulus of the multi-component substance.

### [Operation of system]

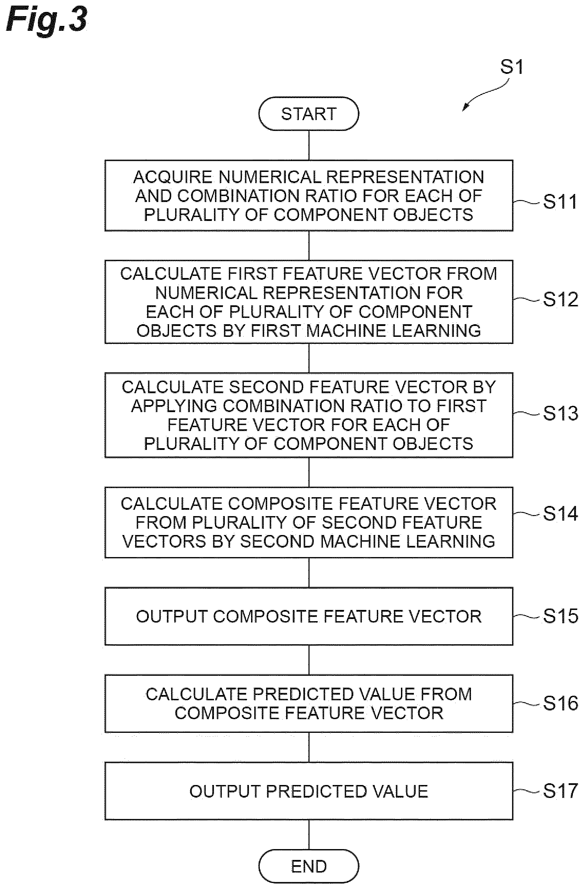

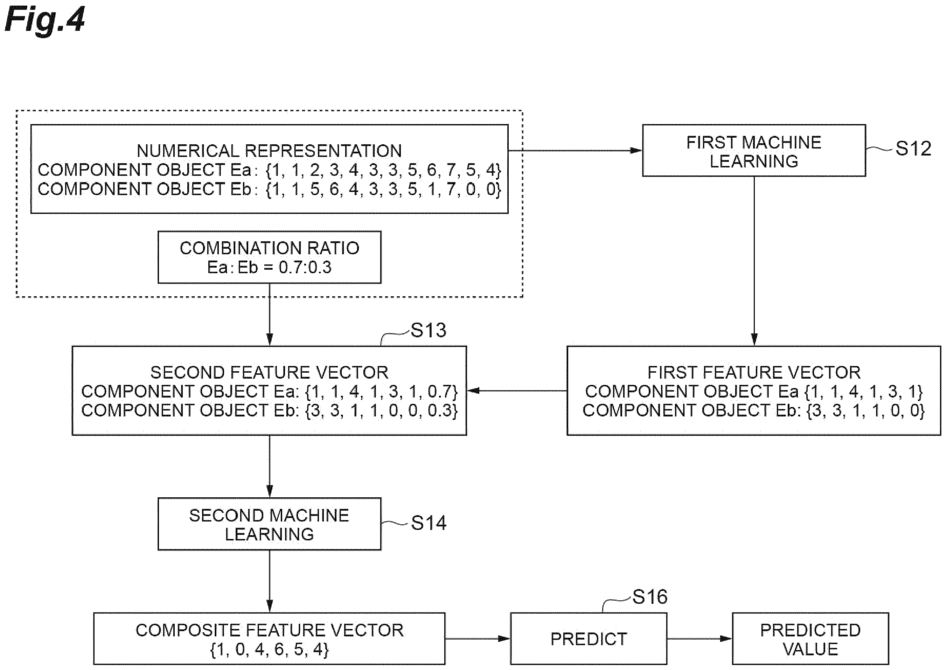

The operation of the information processing system 10 and the information processing method according to the present embodiment will be described with reference to FIGS. 3 and 4. FIG. 3 is a flowchart showing an example of the operation of the information processing system 10 as a processing flow S1. FIG. 4 is a diagram showing the operation of the information processing system 10 by using a specific example of data.

In step S11, the acquisition unit 11 acquires a numerical representation and a combination ratio for each of a plurality of component objects. FIG. 4 shows two component objects Ea and Eb as examples of the plurality of component objects. The numerical representation of the component object Ea is {1, 1, 2, 3, 4, 3, 3, 5, 6, 7, 5, 4}, and the numerical representation of the component object Eb is {1,1, 5, 6, 4, 3, 3, 5, 1, 7, 0, 0}. The combination ratios of the component objects Ea and Eb are 0.7 and 0.3, respectively. This therefore means that the component objects Ea and Eb are used in a ratio of 7:3 to obtain a composite object.

The acquisition unit 11 may acquire the data of each of the plurality of component objects by using any method. For example, the acquisition unit 11 may read data by accessing a given database, or may receive data from another computer or computer system, or may receive data input by the user of the information processing system 10. Alternatively, the acquisition unit 11 may acquire data by any two or more of these methods.

In step S12, the first learning unit 12 calculates a first feature vector from the numerical representation for each of the plurality of component objects by the first machine learning. Specifically, the first learning unit 12 calculates the first feature vector of each of the plurality of component objects by inputting a plurality of numerical representations corresponding to the plurality of component objects into the first machine learning model. In one example, the first learning unit 12 calculates the first feature vector of a component object by inputting the numerical representation corresponding to the component object into the first machine learning model, for each of the plurality of component objects. The first machine learning model is not limited, and may be determined according to an any policy in consideration of factors such as the types of the component object and the composite object. For example, the first learning unit 12 may perform the first machine learning by using the graph neural network (GNN), the convolutional neural network (CNN), or the recurrent neural network (RNN).

In the example of FIG. 4, the first learning unit 12 calculates a first feature vector {1, 1, 4, 1, 3, 1} by inputting the numerical representation {1, 1, 2, 3, 4, 3, 3, 5, 6, 7, 5, 4} of the component object Ea into the first machine learning model. In addition, the first learning unit 12 calculates a first feature vector {3, 3, 1, 1, 0, 0} by inputting the numerical representation {1, 1, 5, 6, 4, 3, 3, 5, 1, 7, 0, 0} of the component object Eb into the first machine learning model.

In step S13, the ratio application unit 13 calculates a second feature vector by applying the combination ratio to the first feature vector for each of the plurality of component objects. "Applying the combination ratio to the first feature vector" refers to a process of changing the first feature vector according to the combination ratio. The application method is not limited. For example, the ratio application unit 13 may perform the application by connecting the combination ratio as an additional component to the first feature vector. Alternatively, the ratio application unit 13 may perform the application by multiplying the combination ratio to each component of the first feature vector or by adding the combination ratio to each component of the first feature vector.

In the example of FIG. 4, the ratio application unit 13 connects the combination ratio as a component to the first feature vector. That is, the ratio application unit 13 calculates a second feature vector {1, 1, 4, 1, 3, 1, 0.7} by connecting the combination ratio 0.7 to the first feature vector {1, 1, 4, 1, 3, 1} of the component object Ea. In addition, the ratio application unit 13 calculates a second feature vector {3, 3, 1, 1, 0, 0, 0.3} by connecting the combination ratio 0.3 to the first feature vector {3, 3, 1, 1, 0, 0} of the component object Eb.

In step S14, the second learning unit 14 calculates a composite feature vector from the plurality of second feature vectors by the second machine learning. In one example, the second learning unit 14 calculates a composite feature vector by inputting a set of a plurality of second feature vectors corresponding to the plurality of component objects into the second machine learning model. The second machine learning model is not limited, and may be determined according to an any policy in consideration of factors such as the types of component objects and composite objects. For example, the second machine learning model may be a learning model that can consider the influence of a combination of elements of the set, or may be a learning model that cannot consider the influence. Examples of the former include the attention RNN and the multi-head attention.

In the example of FIG. 4, the second learning unit 14 calculates a composite feature vector {1, 0, 4, 6, 5, 4} by inputting the second feature vector {1, 1, 4, 1, 3, 1, 0.7} of the component object Ea and the second feature vector {3, 3, 1, 1, 0, 0, 0.3} of the component object Ea into the second machine learning model.

In step S15, the second learning unit 14 outputs the composite feature vector. In the present embodiment, the second learning unit 14 outputs the composite feature vector to the prediction unit 15 for subsequent processing in the information processing system 10. However, the method of outputting the composite feature vector is not limited to this, and may be designed according to an any policy. For example, the second learning unit 14 may store the composite feature vector in a given database, or may transmit the composite feature vector to another computer or computer system, or may display the composite feature vector on a display device.

In step S16, the prediction unit 15 calculates a predicted value of the characteristics of the composite object from the composite feature vector. The prediction method is not limited, and may be designed according to an any policy. For example, the prediction unit 15 may calculate the predicted value from the composite feature vector by the third machine learning. Specifically, the prediction unit 15 calculates a predicted value by inputting the composite feature vector into the third machine learning model. The third machine learning model is not limited, and may be determined according to an any policy in consideration of factors such as the type of the composite object. For example, the prediction unit 15 may perform the third machine learning by using an any neural network to solve a regression problem or a classification problem. Typically, the predicted value of the regression problem is expressed numerically, and the predicted value of the classification problem indicates a category. The prediction unit 15 may calculate the predicted value by using a method other than machine learning.

In step S17, the prediction unit 15 outputs the predicted value. The method of outputting the predicted value is not limited. For example, the prediction unit 15 may store the predicted value in a given database, or may transmit the predicted value to another computer or computer system, or may display the predicted value on a display device. Alternatively, the prediction unit 15 may output the predicted value to another functional element for subsequent processing in the information processing system 10.

### [Program]

An information processing program for causing a computer or a computer system to function as the information processing system 10 includes a program code for causing the computer system to function as the acquisition unit 11, the first learning unit 12, the ratio application unit 13, the second learning unit 14, and the prediction unit 15. The information processing program may be provided after being fixedly recorded on a tangible recording medium such as a CD-ROM, a DVD-ROM, or a semiconductor memory. Alternatively, the information processing program may be provided through a communication network as a data signal superimposed on a carrier wave. The provided information processing program is stored in, for example, the auxiliary storage unit 103. Each of the functional elements described above is realized by the processor 101 reading the information processing program from the auxiliary storage unit 103 and executing the information processing program.

### [Effect]

As described above, an information processing system according to one aspect of the present disclosure includes at least one processor. The at least one processor is configured to: acquire a numerical representation and a combination ratio for each of a plurality of component objects; calculate a first feature vector of each of the plurality of component objects by inputting a plurality of the numerical representations corresponding to the plurality of component objects into a first machine learning model; calculate a second feature vector of each of the plurality of component objects by applying the combination ratio to the first feature vector for each of the plurality of component objects; calculate a composite feature vector indicating features of a composite object obtained by combining the plurality of component objects, by inputting a plurality of the second feature vectors corresponding to the plurality of component objects into a second machine learning model; and output the composite feature vector.

An information processing method according to one aspect of the present invention is executed by an information processing system including at least one processor. The information processing method includes: acquiring a numerical representation and a combination ratio for each of a plurality of component objects; calculating a first feature vector of each of the plurality of component objects by inputting a plurality of the numerical representations corresponding to the plurality of component objects into a first machine learning model; calculating a second feature vector of each of the plurality of component objects by applying the combination ratio to the first feature vector for each of the plurality of component objects; calculating a composite feature vector indicating features of a composite object obtained by combining the plurality of component objects, by inputting a plurality of the second feature vectors corresponding to the plurality of component objects into a second machine learning model; and outputting the composite feature vector.

An information processing program according to one aspect of the present invention causes a computer to execute: acquiring a numerical representation and a combination ratio for each of a plurality of component objects; calculating a first feature vector of each of the plurality of component objects by inputting a plurality of the numerical representations corresponding to the plurality of component objects into a first machine learning model; calculating a second feature vector of each of the plurality of component objects by applying the combination ratio to the first feature vector for each of the plurality of component objects; calculating a composite feature vector indicating features of a composite object obtained by combining the plurality of component objects, by inputting a plurality of the second feature vectors corresponding to the plurality of component objects into a second machine learning model; and outputting the composite feature vector.

In such an aspect, the features of each component object configuring the composite object are clarified before considering the combination ratio. It is therefore possible to improve the accuracy of analysis of composite object even in a case where a sufficient amount of data cannot be prepared for the component object.

In the information processing system according to another aspect, the at least one processor may be configured to apply the combination ratio to the first feature vector by one of connecting the combination ratio to the first feature vector; multiplying the combination ratio to each component of the first feature vector; and adding the combination ratio to each component of the first feature vector. In this case, the combination ratio can be applied to the first feature vector by a simple calculation.

In the information processing system according to another aspect, the at least one processor may be further configured to: calculate a predicted value of characteristics of the composite object by inputting the composite feature vector into the third machine learning model; and output the predicted value. This process makes it possible to accurately calculate the characteristics of the composite object.

In the information processing system according to another aspect, the component object may be a material, and the composite object may be a multi-component substance. In this case, the features of each material configuring the multi-component substance are clarified before considering the combination ratio. It is therefore possible to improve the accuracy of analysis of multi-component substance even in a case where a sufficient amount of data cannot be prepared for the material.

In the information processing system according to another aspect, the material may be a polymer, and the multi-component substance may be a polymer alloy. In this case, the features of each polymer configuring the polymer alloy are clarified before considering the combination ratio. It is therefore possible to improve the accuracy of analysis of polymer alloy even in a case where a sufficient amount of data cannot be prepared for the polymer. There are a huge variety of polymer alloys, and correspondingly, there are a huge variety of polymers. For such polymers and polymer alloys, in general, only some of the possible combinations can be tested, and thus a sufficient amount of data cannot be obtained in many cases. According to this aspect, it is possible to accurately analyze the polymer alloy even in a case where the amount of data is not sufficient as described above.

### [Modifications]

The present invention has been described in detail based on the embodiment. However, the present invention is not limited to the embodiment described above. The present invention can be modified in various ways without departing from its gist.

In the embodiment described above, the information processing system 10 includes the prediction unit 15, but this functional element may be omitted. That is, the process of predicting the characteristics of the composite object may be performed by a computer system different from the information processing system.

The processing procedure of the information processing method executed by at least one processor is not limited to the example in the embodiment described above. For example, some of the steps (processes) described above may be omitted, or the steps may be executed in a different order. In addition, any two or more steps among the above-described steps may be combined, or a part of each step may be modified or deleted. Alternatively, other steps may be executed in addition to each of the above steps. For example, the processing of steps S16 and S17 may be omitted.

In a case of comparing the magnitudes of two numerical values in the information processing system, either of the two criteria of "equal to or greater than" and "greater than" may be used, or either of the two criteria of "equal to or less than" and "less than" may be used. Such criteria selection does not change the technical significance of the process of comparing the magnitudes of two numerical values.

In the present disclosure, the expression "at least one processor performs a first process, performs a second process, ..., and performs an n-th process" or the expression corresponding thereto shows a concept including a case where an execution subject (that is, a processor) of n processes from the first process to the n-th process changes on the way. That is, this expression shows a concept including both a case where all of the n processes are performed by the same processor and a case where the processor is changed according to an any policy in the n processes.

### Reference Signs List

10: information processing system, 11: acquisition unit, 12: first learning unit, 13: ratio application unit, 14: second learning unit, 15: prediction unit.

## Claims

1. An information processing system, comprising:
at least one processor,
wherein the at least one processor is configured to:
acquire a numerical representation and a combination ratio for each of a plurality of component objects;
calculate a first feature vector of each of the plurality of component objects by inputting a plurality of the numerical representations corresponding to the plurality of component objects into a first machine learning model;
calculate a second feature vector of each of the plurality of component objects by applying the combination ratio to the first feature vector for each of the plurality of component objects;
calculate a composite feature vector indicating features of a composite object obtained by combining the plurality of component objects, by inputting a plurality of the second feature vectors corresponding to the plurality of component objects into a second machine learning model; and
output the composite feature vector.

2. The information processing system according to claim 1,
wherein the at least one processor is configured to apply the combination ratio to the first feature vector by one of connecting the combination ratio to the first feature vector; multiplying the combination ratio to each component of the first feature vector; and adding the combination ratio to each component of the first feature vector.

3. The information processing system according to claim 1 or 2,
wherein the at least one processor is further configured to:
calculate a predicted value of characteristics of the composite object by inputting the composite feature vector into a third machine learning model; and
output the predicted value.

4. The information processing system according to any one of claims 1 to 3,
wherein the component object is a material, and the composite object is a multi-component substance.

5. The information processing system according to claim 4,
wherein the material is a polymer, and the multi-component substance is a polymer alloy.

6. An information processing method executed by an information processing system including at least one processor, the method comprising:
acquiring a numerical representation and a combination ratio for each of a plurality of component objects;
calculating a first feature vector of each of the plurality of component objects by inputting a plurality of the numerical representations corresponding to the plurality of component objects into a first machine learning model;
calculating a second feature vector of each of the plurality of component objects by applying the combination ratio to the first feature vector for each of the plurality of component objects;
calculating a composite feature vector indicating features of a composite object obtained by combining the plurality of component objects, by inputting a plurality of the second feature vectors corresponding to the plurality of component objects into a second machine learning model; and
outputting the composite feature vector.

7. An information processing program causing a computer to execute:
acquiring a numerical representation and a combination ratio for each of a plurality of component objects;
calculating a first feature vector of each of the plurality of component objects by inputting a plurality of the numerical representations corresponding to the plurality of component objects into a first machine learning model;
calculating a second feature vector of each of the plurality of component objects by applying the combination ratio to the first feature vector for each of the plurality of component objects;
calculating a composite feature vector indicating features of a composite object obtained by combining the plurality of component objects, by inputting a plurality of the second feature vectors corresponding to the plurality of component objects into a second machine learning model; and
outputting the composite feature vector.
